# EUROPEAN PATENT SPECIFICATION

(11) **EP 3 324 993 B1**
(45) Date of publication and mention of the grant of the patent: **04.09.2024**
(21) Application number: 16828549.2
(22) Date of filing: 21.07.2016
(51) Int. Cl.: A61K 38/06, A61K 33/04, A61P 31/14, A61K 9/127

(54) **LIPOSOMAL REDUCED GLUTATHIONE FOR TREATMENT OF RSV**
LIPOSOMALES REDUZIERTES GLUTATHION ZUR BEHANDLUNG DES RSV
GLUTATHION RÉDUIT LIPOSOMAL POUR LE TRAITEMENT DU VRS

(30) Priority: 21.07.2015 US 201562194874 P; 22.03.2016 US 201662311673 P
(43) Date of publication of application: 30.05.2018
(73) Proprietor: Emory University, Atlanta, Georgia 30322 (US); Your Energy Systems, LLC, Palo Alto, CA 94301 (US)
(72) Inventor: BROWN, Lou, Ann, Atlanta, GA 30322 (US); GUILFORD, Frederick, Timothy, Palo Alto, CA 94301 (US)
(74) Representative: Isarpatent
(86) International application number: PCT/US2016/043372
(87) International publication number: WO 2017/015481

(56) References cited:
- JP-A- 2005 089 450
- KR-A- 20130 122 018
- US-A1- 2011 129 523
- US-A1- 2012 244 212
- MANUEL MATA ET AL: "Respiratory Syncytial Virus Inhibits Ciliagenesis in Differentiated Normal Human Bronchial Epithelial Cells: Effectiveness of N-Acetylcysteine", PLOS ONE, vol. 7, no. 10, 31 October 2012 (2012-10-31), pages e48037, XP055554299, DOI: 10.1371/journal.pone.0048037
- ROBERTO P. GAROFALO ET AL: "Respiratory Syncytial Virus Infection: Mechanisms of Redox Control and Novel Therapeutic Opportunities", ANTIOXIDANTS AND REDOX SIGNALING, vol. 18, no. 2, 10 January 2013 (2013-01-10), US, pages 186 - 217, XP055554297, ISSN: 1523-0864, DOI: 10.1089/ars.2011.4307
- COOKE RICHARD W I ET AL: "Reduction of oxidative stress marker in lung fluid of preterm infants after administration of intra-tracheal liposomal glutathione", BIOLOGY OF THE NEONATE, KARGER, BASEL, CH, vol. 87, no. 3, April 2005 (2005-04-01), pages 178 - 180, XP009511113, ISSN: 0006-3126, [retrieved on 20050406], DOI: 10.1159/000082623
- FRATERNALE A ET AL: "GSH and analogs in antiviral therapy", MOLECULAR ASPECTS OF MEDICINE, PERGAMON PRESS, OXFORD, GB, vol. 30, no. 1-2, February 2009 (2009-02-01), pages 99 - 110, XP026096212, ISSN: 0098-2997, [retrieved on 20080927], DOI: 10.1016/J.MAM.2008.09.001
- DATABASE MEDLINE [online] US NATIONAL LIBRARY OF MEDICINE (NLM), BETHESDA, MD, US; November 1997 (1997-11-01), LIU X ET AL: "[Effects of selenium supplement on acute lower respiratory tract infection caused by respiratory syncytial virus].", XP002788784, Database accession no. NLM9863072
- MEJIAS ASUNCION ET AL: "New options in the treatment of respiratory syncytial virus disease", JOURNAL OF INFECTION, vol. 71, 25 April 2015 (2015-04-25), XP029231667, ISSN: 0163-4453, DOI: 10.1016/J.JINF.2015.04.025
- MOCHIZUKI ET AL.: "RS virus-induced inflammation and the intracellular glutathione redox state in cultured human airway epithelial cells", INFLAMMATION, vol. 32, no. 4, 2009, pages 252 - 264, XP019729884

## Description

### TECHNICAL FIELD

This invention relates to a composition for use in a method of treatment of Respiratory Syncytial Virus (RSV) infections. The composition for use in treatment is a liposomally formulated reduced glutathione.

### BACKGROUND

Respiratory syncytial virus is a virus from the family Paramyxoviridae, which includes common respiratory viruses, and is a member of the paramyxovirus subfamily Pneumovirinae. Its name comes from the fact that F proteins on the surface of the virus cause the cell membranes on nearby cells to merge, forming syncytia. It causes a viral type of pneumonia symptoms or symptoms of bronchiolitis. It is highly transmissible.

The Centers for Disease Control describes RSV as follows:

### "In Infants

RSV infection can cause a variety of respiratory illnesses. And these illnesses sometimes cause fever. RSV infection most commonly causes a cold-like illness. But it can also cause bronchitis, croup, and lower respiratory infections like bronchiolitis and pneumonia. Of every 100 infants and young children with RSV infection, 25 to 40 (25% to 40%) will show signs of pneumonia or bronchiolitis. Premature infants, very young infants, and those with chronic (always present) lung or heart disease or with suppressed (weakened) immune systems have a greater chance of having a more severe infection (such as a lower respiratory tract infection). Infection without symptoms is rare among infants.

Infants with a lower respiratory tract infection typically have a runny nose and a decrease in appetite before any other symptoms appear. Cough usually develops 1 to 3 days later.

Soon after the cough develops, sneezing, fever, and wheezing may occur. In very young infants, irritability, decreased activity, and apnea may be the only symptoms of infection.

Most otherwise healthy infants who are infected with RSV do not need hospitalization. Those who are hospitalized may require oxygen, intubation, and/or mechanical ventilation. Most improve with supportive care and are discharged in a few days.

### In Adults

Symptomatic RSV infections may occur in adults, particularly in healthcare workers or caretakers of small children. Disease usually lasts less than 5 days, and symptoms are usually consistent with an upper respiratory tract infection and can include a runny nose (rhinorrhea), sore throat (pharyngitis), cough, headache, fatigue, and fever, but some high-risk adults, such as those with certain chronic illnesses or immunosuppression, may have more severe symptoms consistent with a lower respiratory tract infection, such as pneumonia."
www(dot)cdc.gov/rsv/clinical/description.html downloaded on November 18, 2014.

There is no treatment other than the passage of time and palliative measures according to the United States Centers for Disease Control:
"Q: What is RSV?
A: RSV is the most common cause of bronchiolitis (inflammation of the small airways in the lung) and pneumonia in children under 1 year of age in the United States. Each year, 75,000 to 125,000 children in this age group are hospitalized due to RSV infection. Almost all children are infected with the virus by their second birthday, but only a small percentage develop severe disease."
"Q: How can I provide care to someone with RSV?
A: *There is no specific treatment for RSV infection.* However, there are simple ways to help relieve some of the typical symptoms. Your doctor can give advice on how to make people with RSV infection more comfortable and assess whether hospitalization is needed. [emphasis added]"

From the Centers for Disease Control website downloaded from
www(dot)cdc(dot)gov//rsv/about/faq.html on November 18, 2014

A leading clinic, the Mayo Clinic, based in Rochester Minnesota, has this to say about treatment

### "Treatments and drugs

### By Mayo Clinic Staff

Treatment for respiratory syncytial virus generally involves self-care measures to make your child more comfortable (supportive care). But in severe cases, hospital care may be needed.

### Supportive care

Your doctor may recommend an over-the-counter medication such as acetaminophen (Tylenol, others) to reduce fever. He or she may also prescribe an antibiotic if there's a bacterial complication, such as bacterial pneumonia.

Otherwise, keep your child as comfortable as possible. Offer plenty of fluids and watch for signs of dehydration, such as dry mouth, little to no urine output, sunken eyes and extreme fussiness or sleepiness.

### Hospital care

Hospital care for RSV in severe cases may be necessary to provide intravenous (IV) fluids and humidified oxygen. Hospitalized infants and children may also be hooked up to mechanical ventilation - a breathing machine - to ease breathing.

In some severe cases, a nebulized bronchodilator such as albuterol (ProAir HFA, Proventil-HFA, Ventolin HFA) may be used to relieve wheezing. This medication opens air passages in the lungs. Nebulized means it's administered as a fine mist that you breathe in.

Occasionally, a nebulized form of ribavirin (Virazole), an antiviral agent, may be used. Your doctor may also recommend an injection of epinephrine or a form of epinephrine that can be inhaled through a nebulizer (racemic epinephrine) to relieve symptoms of RSV infection."
www(dot)mayoclinic.org/diseases-conditions/respiratory-syncytial-virus/ basics/treatment/con-20022497 downloaded on November 18, 2014

The National Library of Medicine has this to say:

### "Treatment

Antibiotics do not treat RSV.

Mild infections go away without treatment.

Infants and children with a severe RSV infection may be admitted to the hospital.

Treatment will include:
Oxygen
Moist (humidified) air
Fluids through a vein (by IV)

A breathing machine (ventilator) may be needed."
www(dot)nlm.nih.gov/medlineplus/ency/article/001564.htm downloaded Nov. 18, 2014.

All of these measures referenced by the Mayo Clinic and the National Library of Medicine are directed to alleviation of symptoms, but there is no generally accepted treatment per se.

The American Academy of Pediatrics has the following caution regarding on recommended method of prophylaxis:
**"From the American Academy of Pediatrics**
**Policy Statement**
**Updated Guidance for Palivizumab Prophylaxis Among Infants and Young Children at Increased Risk of Hospitalization for Respiratory Syncytial Virus Infection**
**COMMITTEE ON INFECTIOUS DISEASES AND BRONCHIOLITIS GUIDELINES COMMITTEE....**
**Lack of Therapeutic Efficacy of Palivizumab**
Palivizumab is a humanized monoclonal antibody (IgG) directed against an epitope in the A antigenic site of the F protein of RSV. Passive antibody administration is not effective in treatment of RSV disease and is not approved or recommended for this indication.
**Prevention of Health Care-Associated RSV Disease**
No rigorous data exist to support palivizumab use in controlling outbreaks of health care-associated disease, and palivizumab use is not recommended for this purpose. Infants in a neonatal unit who qualify for prophylaxis because of CLD, prematurity, or CHD may receive the first dose 48 to 72 hours before discharge to home or promptly after discharge.
Strict adherence to infection-control practices is the basis for reducing health care-associated RSV disease"

The patient populations proposed are very limited-palivizumab is not generally recommended. http://pediatrics(dot)aappublications.org/content/134/2/415.full downloaded on Nov. 18, 2014. In sum, there is no generally recommended treatment useful to a broad base of patients to cure RSV.

Manuel Mata el al. (PLOS ONE, 2012-10-31, vol. 7, no. 10, page e48037) describe that N-acetylcysteine (NAC) restores the normal functions in cell cultures of the epithelium that had been affected by the RSV infection.

Roberto P. Garofalo et al. (Antioxidants and Redox Signaling, 2013-01-10, vol. 18, no. 2, pages 186-217) describe that RSV infection induces significant oxidative stress in vitro as well as in vivo: a progressive increase in lipid peroxidation products and a progressive reduction of the GSH-glutathione disulfide ratio in RSV-infected airway epithelial cells. The article suggests only that NAC ought to be helpful to treat symptoms of influenza-induced respiratory symptoms in humans, but also references mixed results using NAC.

Mochizuki et al. (Inflammation, 2009, vol. 32, no. 4, pages 252-264) describe oxidative stress as a potential cause of RSV-induced asthma. Measurements were made of the levels of oxidative stress and appeared to establish that glutathione redox balance was affected by RSV but noted that after the stress, there was a prolonged reduced state. Glutathione monoethyl ester was used as a modulator to show that cytokines and chemokines were affected. Certain cytokines were affected, but many others typically involved in inflammation were unaffected including IL-lbeta, IL-2, IL-5, IL-7, IL-10, IL-12, IL-13, IL-17, MSP-1, MIP1betaS, GM-CSF and TNF-alpha. There was no mention of using glutathione or reduced glutathione to affect the progression of the underlying disease of RSV or affect the virus. No use of the formulation of the present description is suggested or mentioned.

Cooke Richard W I et al. (Biology of The Neonate, Karger, Basel, CH, 2005-04, vol. 87, no. 3, pages 178-180) describe that intratracheal administration of liposomal GSH offers a method of prolonging GSH levels in the lung in pre-term infants.

US-A-2012244212 describes the advantages of using liposomal GSH over GSH and the treatment of HIV+ tuberculosis patients. RSV is not mentioned in the application published. Treatment of the lung itself is not discussed. The dramatic effects shown in the present description on a virus such as RSV are not referenced.

### SUMMARY OF INVENTION

### TECHNICAL PROBLEM

The technical problem is to find a non-toxic treatment with minimal side effects that is useful in a broad patient population to treat RSV

### OBJECTIVES OF THE INVENTION

The inventors propose a composition for use in a method of treatment of RSV according to claim 1. The composition for use in treatment is a liposomally formulated reduced glutathione.

The inventors propose a composition of liposomally formulated reduced glutathione for use in a treatment of RSV infections in immunocompromised individuals, according to claim 3.

### SOLUTION TO PROBLEM

The proposed solution is a composition for use according to claim 1. In said composition for use, the liposomal reduced glutathione can be administered to individuals with viral illness RSV. Liposomal reduced glutathione distributed and sold by Your Energy Systems of Palo Alto, California, can be provided in various forms. The original formulation of liposomal reduced glutathione (LRG) is designed to be taken orally and provide rapid absorption to the body and the lungs. Liposomal reduced glutathione can also be administered intravenously in the liquid form or can be formulated to be dry powder form of liposomes containing reduced glutathione that is designed to be used for reconstitution with a sterile fluid such as water and used for intravenous or pulmonary administration by inhalation therapy. Liposomally formulated reduced glutathione can also be administered mucosally by oral, intranasal, pulmonary routes or into the gastrointestinal tract, including the stomach. Liposomally formulated reduced glutathione is described in Guilford, U.S. Patents 8,252,325, 8,349,359, 8,147,869, and 8,679,530 which are adopted by reference and methods of manufacture in Keller, U.S. Patents 5,891,465 and 6,610,322 are adopted by reference.

Powdered liposomal glutathione is prepared using a modified dehydration-rehydration method. One method for forming powdered liposomal glutathione includes The dry powders containing glutathione-loaded liposomes (SD-reduced glutathione-Lip) were produced following the spray-drying technique and using lactose at 10% (w/v) as drying adjuvant (Marchiori et al., 2012). Then, 10 g of lactose were dispersed in 100 mL of liposomal PBS dispersion (1 mg mL1 of the drug or blank) under magnetic stirring for 10 min. This dispersion was dried using a Mini Spray Dryer B-290 (Büchi, Switzerland) according to the following parameters: feed pump rate of 5.0 mL min⁻¹, 100% aspiration, 0.7 mm nozzle, atomization air at 819 L⁻¹ h inlet temperature 120 °C with an outlet temperature around 65 C.

### Advantageous Effects of Invention

The invention has been recently shown in unpublished animal studies to be effective in significantly reducing the replication of RSV in lung tissues.

### Brief Description of Drawings

The drawings are diagrammatic presentations of color spectrophotographs of mice pups.

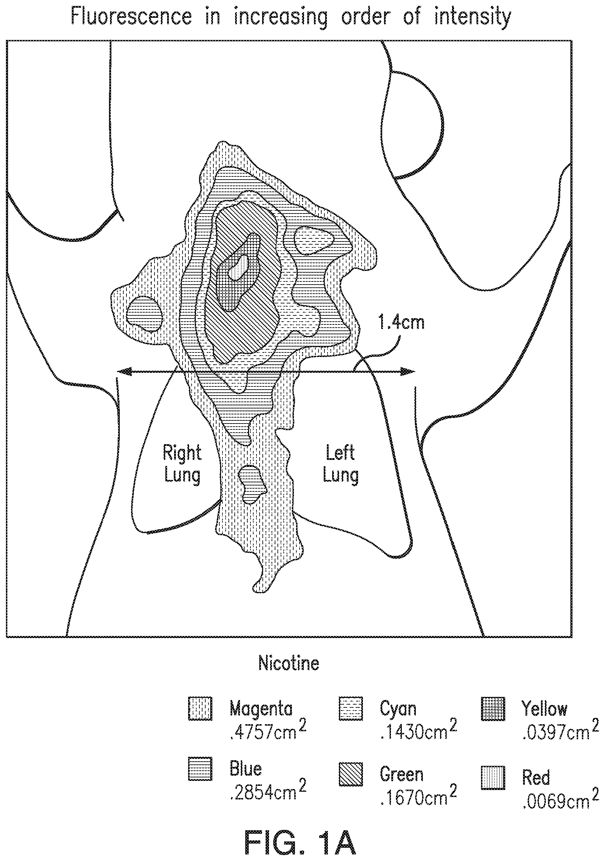

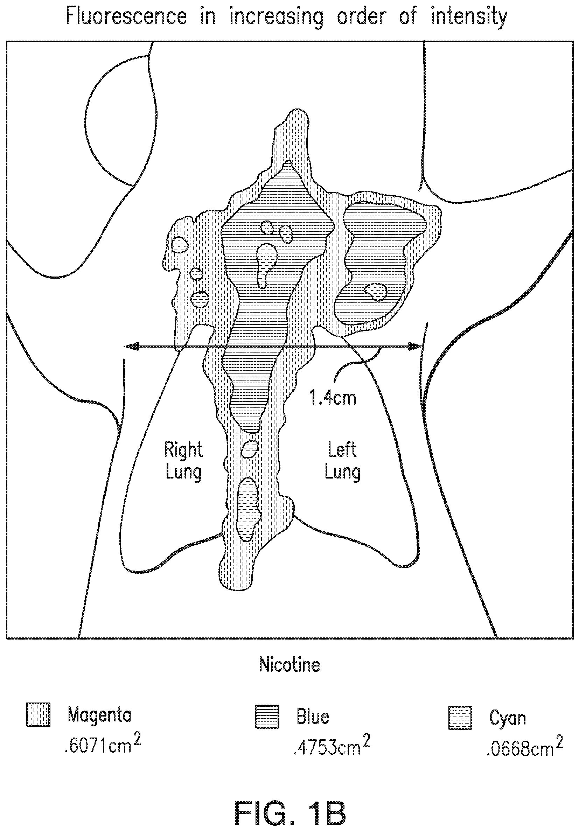

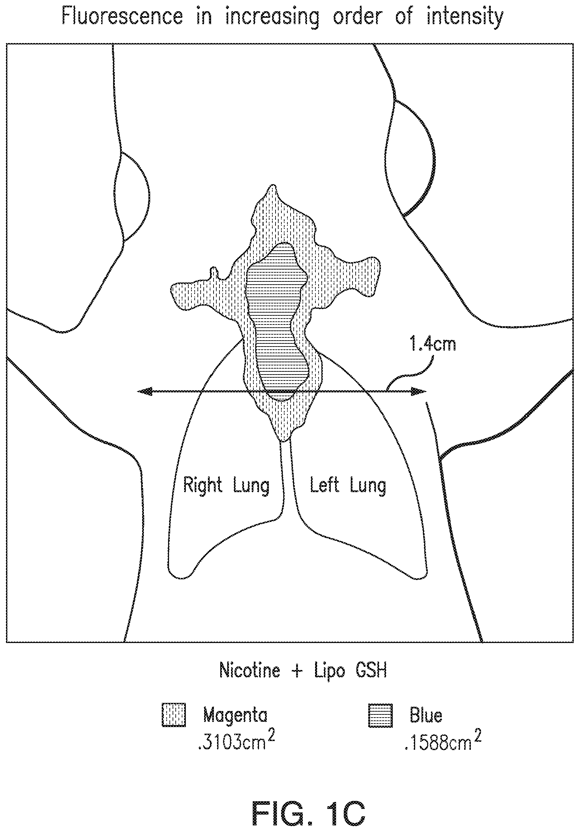

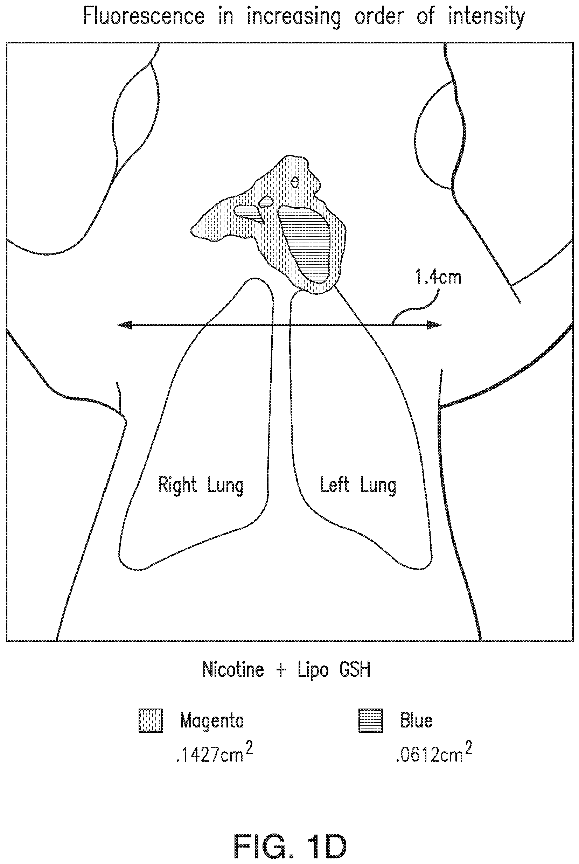

Figures 1A , 1B, 1C, and 1D depict mouse pups exposed to nicotine *in utero* through the addition of nicotine to the drinking water of the pregnant dam mouse. In this model, the nicotine crosses the placenta and the pups are born with decreased GSH in the lungs. The controls are in Figures 1A and 1B. At 10 days of life, two nicotine-exposed pups (Figures 1A and 1B) were gavaged (in the stomach)with saline. In addition, two nicotine-exposed pups (Figures 1C and 1D) were gavaged with LRG. After each respective gavage at 10 days of life, there was the intra-tracheal delivery of an RSV that fluoresces when integrated into the genome of a cell - indicating an RSV- infected cell. The RSV incubates approximately 24 hours and then the spectrophotographs are taken.
   The accompanying drawings depict the original spectrophotographs of the fluorescing RSV in a ventral (front side) view of the animals taken of the animals after incubation of the virus. In the accompanying drawings, the concentration of RSV fluorescence is depicted with an area of intense concentration of fluorescing RSV from the photographs. In Figure 1A, for a GSH-depleted mouse as a result of nicotine, after being treated with only saline, the area of most active infection is shown by red in the center, and then in descending order of intensity: by yellow, then by green, then by cyan, then by blue and finally by magenta. Red is represented by vertical lines, yellow is represented by crosshatch lines, green is represented by lines from upper left to lower right, cyan is represented by dotted horizontal lines, blue is represented by horizontal continuous lines, and magenta is represented by dotted vertical lines.
Figure 1B is a view of a second animal also treated with only saline. That animal is slightly less but also severely infected.
Figure 1C shows an animal gavaged with liposomally formulated glutathione. A much smaller area is infected with RSV, and a much smaller area of the lung. Further, the absolute level of infection, indicated by reduced fluorescence, shows only magenta and blue and no level of higher intensity (red, yellow, green or cyan) of infection as seen in Figures 1A and 1B.
Figure 1D portrays experimental data for a second mouse gavaged with liposomally formulated glutathione with similar dramatic results compared to Figures 1A and 1B.
   The accompanying graphic drawings depict a significant reduction of infection with RSV in the lungs of animals administered LRG. The areas for each color have been calculated assuming an approximate armpit to armpit distance of 1.4 cm.
   These are the photographs diagrammatically depicted in Figures 1A through 1D, respectively.

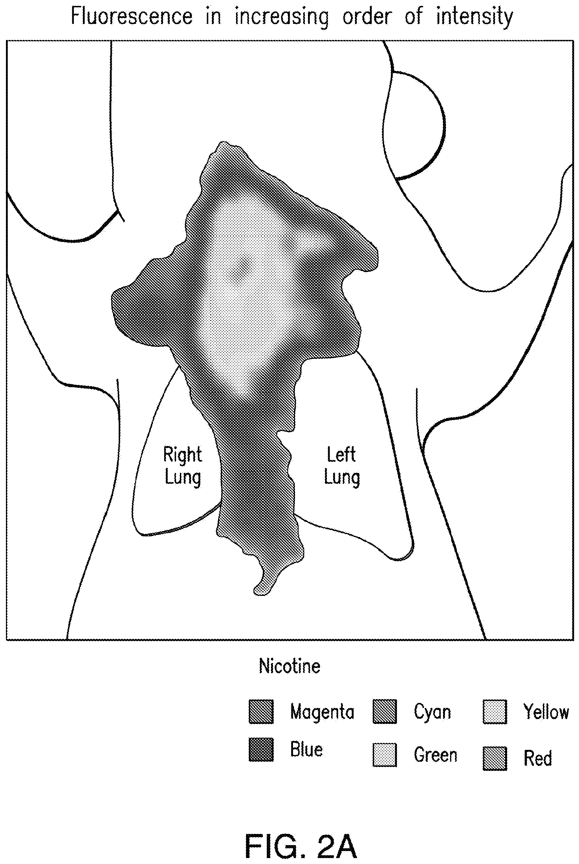

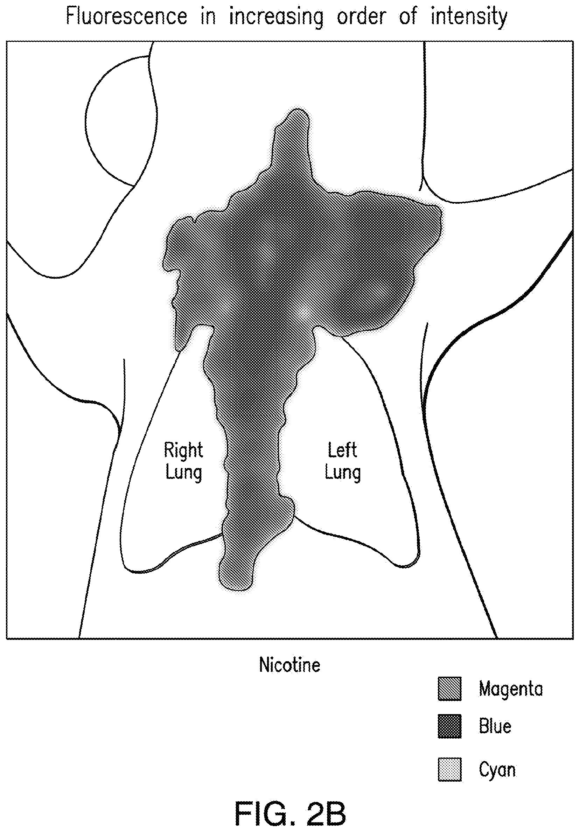

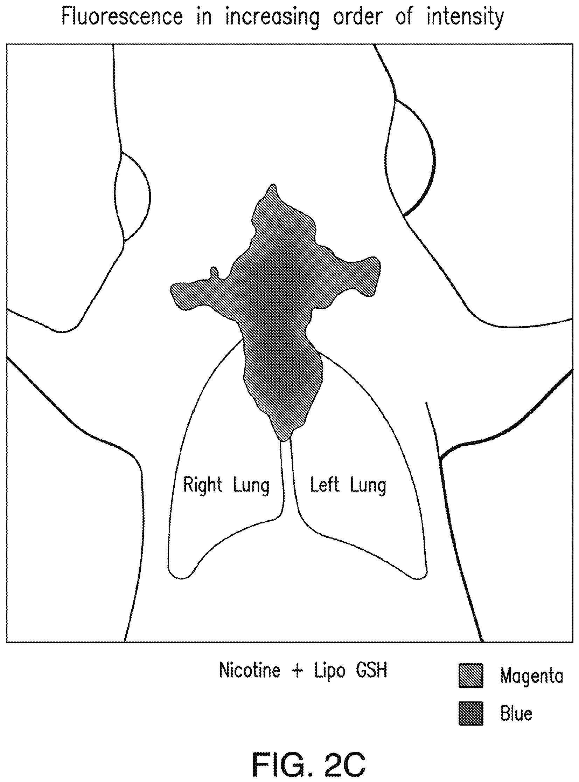

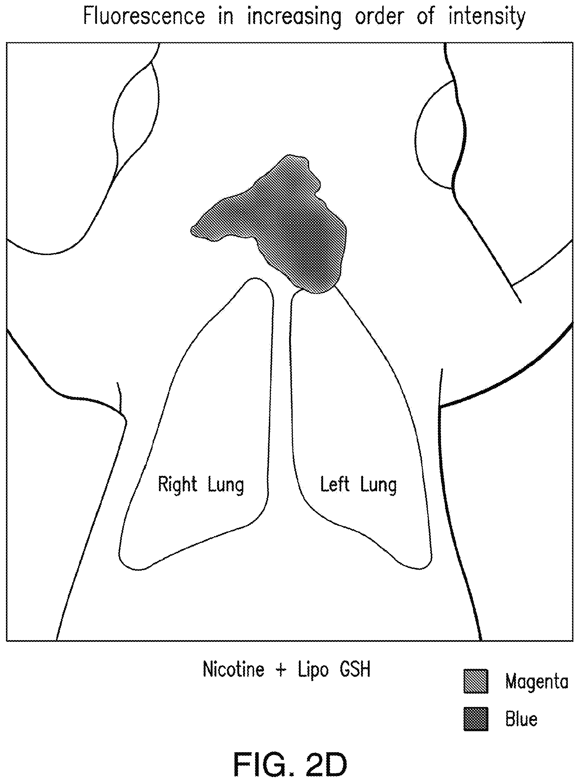

Figures 2A through 2D are color photographs converted to grayscale corresponding to the colors reflecting intensity of infection superimposed on the mouse pup diagram; red is so intense that it shows as a darker spot in the center of the white area in the original photographs.

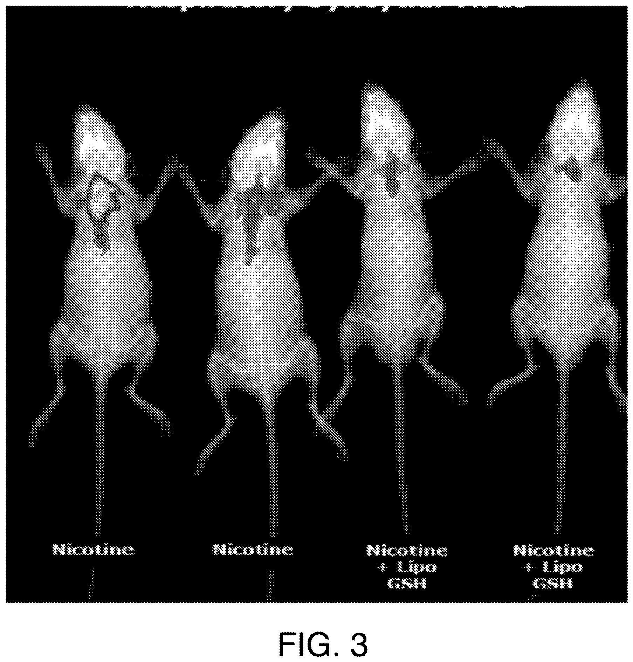

Figures 3 are the color fluorescent photos showing the reduction in the GSH treated mice pups versus the controls.

### Description of Embodiments

### Examples

- In the following examples, the physician is aware that by age 2 years, most children have been infected with RSV at least once (1). The CDC website on RSV (2) shows that 25 to 40 out of 100 of them have signs or symptoms of bronchiolitis or pneumonia, and
- 5 to 20 out of 1,000 will require hospitalization. Most children hospitalized for RSV infection are younger than 6 months of age.
Infants and children infected with RSV usually show symptoms within 4 to 6 days of infection. Most will recover in 1 to 2 weeks. However, even after recovery, very young infants and children with weakened immune systems can continue to spread the virus for 1 to 3 weeks.

Reduced liposomal glutathione or reduced liposomally formulated glutathione can be produced according to the techniques in Guilford, U.S. Pat. 8,252,325, 8,147,869 and 8,679,530. The product proposed for treatment herein is sold by Your Energy Systems, LLC, 555 Bryant St., Suite 305, Palo Alto, CA 49301

Selenium 200 mcg may be used in addition to LRG.

### Example 1

A 15 month old child weighing 30 pounds (13.6 kg) presents to the physician in a hospital emergency room with symptoms of infection involving the lungs that have been present for 24 hours and examination sounds to the physician as bronchiolitis. A nasal wash sample is collected for confirmatory diagnostics using viral culture, or detection of viral antigens or RNA sequences (3). A blood sample for serologic detection of antibody to RSV may also be obtained. In this case, the rapid RSV antigen test is run on the sample obtained and will be available in 1.5 hours. After the examination and with the symptoms consistent with viral infection of the lung, the physician knows that the majority of pulmonary infections in this age group are RSV related. With the diagnosis of viral infection, likely to be RSV related, the physician elects to initiate administration of oral LRG. The dosing schedule for non-acute, chronic conditions is ¼ teaspoon RLG (containing 100 mg glutathione) per for every 30 pounds (13.6 kg) of weight. As this is an acute infection, the physician elects to initiate therapy with ½ teaspoon of RLG for every 30 pounds (13.6 kg) using oral administration. The ½ teaspoon is continued every 12 hours for 14 days. The laboratory test subsequently confirms the clinical diagnosis of RSV infection.

### Example 2

An elderly adult lives in a house with a young child who is experiencing a viral upper respiratory infection, presents to the emergency department with symptoms of a cough for 24 hours. The diagnosis of RSV is suspected as there is no sign of bacterial infection. Serologic testing is obtained but will take 2 days to become available. Rapid antigen testing is less efficient in this setting as only nasal swab can be obtained. The physician elects to initiate treatment with liposomal reduced glutathione. The dosing for adults with a chronic condition is 1 teaspoon (420 mg glutathione) twice a day. In an acute situation such as this additional doses may be considered and the physician recommends 2 teaspoons every 6 hours for 24 hours to support glutathione, which may be reduced in this condition. It is known that plasma glutathione levels are decreased in aging (4). The serologic test for RSV subsequently retuned positive.

### Dosing

DETERMINE INDIVIDUAL DOSE BY BODY WEIGHT: For children
Under 30 lbs (13.6 kg): ¼ - ½ teaspoon = 100 - 200 mg GSH
30 - 60 lbs (13.6 - 27.2 kg): ½ - 1 teaspoon = 210 - 420 mg GSH
60 - 90 lbs (27.2 - 40.8 kg): 3/4 - 1.5 teaspoon = 316 mg - 630 GSH
90 - 120 lbs (40.8 - 54.4 kg): 1 -2 teaspoons = 422 - 844 mg GSH
120 - 150 lbs (54.4 - 68.0 kg): 1 ½ - 3 teaspoons = 630 - 1260 mg GSH
Over 150 lbs (68.0 kg): 1 ½ - 3 teaspoons = 630 - 1260 mg GSH

The invention should be used on a continuous basis.

Children - should use a dose of liposomally encapsulated reduced glutathione equivalent to 60 mg/Kg of body weight daily in divided doses.

These doses should be continued for the duration of the duration of the illness and for prevention of recurrence and to decrease the chance of shedding of virus for 2-3 weeks after the illness Respiratory syncytial virus.

The components of this invention can be administered separately or combined in a single capsule or dose.

As RSV is more likely to occur in elderly or in adult individuals with compromise of the immune system, an increased dose from the standard glutathione support dose is recommended in the elderly population for acute conditions.

For standard support 1 teaspoon twice a day is recommended. For management of RSV a dose of 2 teaspoons twice a day for 2 - 3 weeks is recommended. After that time, if the individual is suspected to have decreased glutathione and/or immune compromise a dose of 2 teaspoons twice a day may be continued.

Immunocompromised individuals are at even higher risk. Immunocompromised means individuals who are HIV positive or already have AIDS-related complex (ARDC). It also means individuals with tuberculosis, and individuals who are receiving any radiation or chemotherapeutic treatment, or who display any immune disorder. For these individuals in this paragraph, the recommended dosing is the same as for the elderly population for acute conditions.

### INDUSTRIAL APPLICABILITY

The inventors propose a composition for use in a method of treatment of RSV infections, wherein the composition for treatment is a liposomally formulated reduced glutathione.

### Citation List

### Non Patent Literature

1. Moyes J, Cohen C, Pretorius M, Groome M, von Gottberg A, Wolter N, et al. Epidemiology of Respiratory Syncytial Virus-Associated Acute Lower Respiratory Tract Infection Hospitalizations Among HIV-Infected and HIV-Uninfected South African Children, 2010-2011. Journal of Infectious Diseases. 2013;208(suppl 3):S217-S26. http://jid.oxfordjournals.org/content/208/suppl_3/S217.abstract
2. CDC. RSV - Respiratory Syncytial Virus Infection Infection and Incidence. CDC 24/7: Saving Lives, Protecting People [serial on the Internet]. 2014 Available from: http://www.cdc.gov/rsv/about/infection.html.
3. Falsey AR, Formica MA, Walsh EE. Diagnosis of respiratory syncytial virus infection: comparison of reverse transcription-PCR to viral culture and serology in adults with respiratory illness. J Clin Microbiol. 2002;40(3):817-20. PMCID: 120281. http://www.ncbi.nlm.nih.gov/pubmed/11880399
4. Lang CA, Naryshkin S, Schneider DL, Mills BJ, Lindeman RD. Low blood glutathione levels in healthy aging adults. J Lab Clin Med. 1992;120(5):720-5. http://www.ncbi.nlm.nih.gov/entrez/query.fcgi?cmd=Retrieve&db=PubMed&dopt=Citation&list _uids=1431500

## Claims

1. A composition of liposomal reduced glutathione for use in a method of treatment of Respiratory Syncytial Virus infections.

2. The composition for use according to claim 1 comprising liposomal reduced glutathione and selenium.

3. The composition for use according to claim 1 or 2 for use in a method of treatment of Respiratory Syncytial Virus infections in an immunocompromised individual.

## Patentansprüche

1. Zusammensetzung aus liposomalem reduziertem Glutathion zur Verwendung in einem Verfahren zur Behandlung von Infektionen mit dem Respiratorischen Synzytial-Virus.

2. Zusammensetzung zur Verwendung nach Anspruch 1, die liposomales reduziertes Glutathion und Selen enthält.

3. Zusammensetzung zur Verwendung nach Anspruch 1 oder 2 zur Verwendung in einem Verfahren zur Behandlung von Infektionen mit dem Respiratorischen Synzytial-Virus bei einer immungeschwächten Person.

## Revendications

1. Composition de glutathion réduit liposomal destinée à l'utilisation dans un procédé de traitement d'infections à virus respiratoire syncytial.

2. Composition pour une utilisation selon la revendication 1, comportant du glutathion réduit liposomal et du sélénium.

3. Composition pour l'utilisation selon la revendication 1 ou 2 destinée à l'utilisation dans un procédé de traitement d'infections à virus respiratoire syncytial chez un individu immunodéprimé.
